# EUROPEAN PATENT APPLICATION

(11) **EP 4 011 386 A1**
(43) Date of publication of application: **15.06.2022**
(21) Application number: 21218063.2
(22) Date of filing: 31.01.2018
(51) Int. Cl.: A61K 38/09, A61P 35/00, A61K 9/00, A61K 9/06, A61K 9/16, A61K 47/34

(54) **COMPOSITIONS AND METHODS FOR LONG TERM RELEASE OF GONADOTROPIN-RELEASING HORMONE (GNRH) ANTAGONISTS**

(30) Priority: 31.01.2017 US 201762452788 P
(62) Divisional of application: 18747581.9
(71) Applicant: Veru Inc., Miami, Florida 33137 (US)
(72) Inventor: KACKER, Ravi, Lexington, MA 02421 (US); STEINER, Mitchell S., Germantown, TN 2600 (US)
(74) Representative: Kutzenberger Wolff & Partner

(57) **Abstract**

The invention provides compositions and methods for long term release of Gonadotropin-releasing hormone (GnRH) antagonists, and uses thereof. Specifically, the invention provides polymer compositions and methods for controlled release of GnRH antagonists.

## Description

### CROSS-REFERENCE TO RELATED APPLICATIONS

This application claims priority to and the benefit of United States Provisional Patent Application 62/452,788, filed January 31, 2017, which is incorporated by reference herein in its entirety.

### FIELD OF THE INVENTION

The invention relates to compositions and methods for long term release of Gonadotropin-releasing hormone (GnRH) antagonists, and uses thereof. Specifically, the invention relates to polymer based compositions and methods for controlled release of GnRH antagonists.

### BACKGROUND OF THE INVENTION

The hypothalamic hormone, gonadotropin-releasing hormone (GnRH) (also known as luteinizing hormone releasing hormone (LHRH)), controls the secretion of the gonadotropins, luteinizing hormone (LH) and follicle stimulating hormone (FSH) from the anterior pituitary gland. GnRH is secreted by the hypothalamus, and stimulates secretion of luteinizing hormone (LH) and follicle stimulating hormone (FSH). Analogues of GnRH are currently used to treat many medical conditions that require manipulation of the production of the sex hormones, testosterone and estrogen. Schally et al. (*Schally* 1971) isolated, identified the amino acid sequence, and synthesized the peptide hormone GnRH. Deletion or replacement of different amino acids of GnRH peptide has resulted in the discovery of GnRH agonist analogues that demonstrate greater potency for the secretion of LH and FSH. A paradoxical clinical effect occurs when agonistic analogues are used continuously such that after the chronic, and relatively long period (2-3 weeks) of stimulation of the secretion of LH and FSH, there is actually an inhibition of LH and FSH release and consequent suppression of sex steroid production. (*Reissmann 2000).* In certain medical conditions, however, an immediate and dose-dependent suppression of LH and FSH is desired. Over 20 years ago, Schally and Revier synthesized the 1^{st} generation analogues of GnRH antagonist analogues which were too lipophilic and induced histamine release. (*Schmidt 1984; Hahn 1985).* The 2^{nd} generation GnRH antagonist analogues were made by incorporating further amino acid substitutions (*Bajusz 1988; Rivier 1993)* that resulted in potentially safer and more effective decapeptide analogues. Examples of newer generation GnRH antagonist analogues include abarelix, degarelix, ganirelix, ozarelix, cetrorelix, taverelix, antarelix, and iturelix.

Clinical development and medical applications of these GnRH antagonist analogues have been either successful or attempted for controlled ovarian stimulation for assisted reproductive techniques, uterine myoma, ovarian cancer, benign prostatic hyperplasia, and prostate cancer. In certain diseases and conditions, the major limitation for successful application of the GnRH antagonist analogue has been having only a short acting formulation where longer acting depot formulations would be more advantageous.

Accordingly, there exists a need for very long acting controlled or extended release formulations of a GnRH antagonist (also called a LHRH antagonist).

### SUMMARY OF THE INVENTION

In one aspect, the invention relates to a composition, said composition comprising: a therapeutically effective amount of a GnRH antagonist in combination with a polymer, wherein said polymer is poly(glycolide) (PLG), poly(lactide) (PLA), or poly-lactic co-glycolic acid (PLGA), wherein said composition is capable of releasing said GnRH antagonist for a long term (e.g., more than 90 days). In an exemplary embodiment, GnRH antagonist is cetrorelix, abarelix, degarelix, ganirelix, ozarelix, taverelix, antarelix, or iturelix.

In another aspect, the invention relates to a composition, said composition comprising: a therapeutically effective amount of a GnRH antagonist in combination with a non-PLGA block polymer, wherein said polymer is polyethyleneglycol (PEG), PLG, PLA, polybutylene terephthalate (PBT), poly(epsilon-caprolactone) (PCL), dioxanone, butanediisocyanate, butanediol, polyoxyetylene, polypropylene, polyoxypropylene, polystyrene, poly methyl methacylate, or a combination thereof, wherein said composition is capable of releasing said GnRH antagonist for a long term.

In another aspect, the invention relates to a flowable composition, the composition comprising: (a) a biodegradable thermoplastic polyester that is at least substantially insoluble in aqueous medium or body fluid; (b) a biocompatible polar aprotic solvent, wherein the biocompatible polar aprotic solvent is miscible to dispersible in aqueous medium or body fluid; and (c) a therapeutically effective amount of a GnRH antagonist. In an exemplary embodiment, the thermoplastic polyester is a polylactide, a polyglycolide, a polycaprolactone, a copolymer thereof, a terpolymer thereof, or any combination thereof. In another exemplary embodiment, the solvent is N-methyl-2-pyrrolidone, 2-pyrrolidone, N,N-dimethylformamide, dimethyl sulfoxide, propylene carbonate, caprolactam, triacetin, or any combination thereof. In a particular embodiment, the flowable composition of the invention comprises a flowable delivery system such as an Atrigel^{®} system comprising a copolymer, a water soluble organic solvent, and a bioactive agent, for example, a GnRH antagonist.

In another aspect, the invention relates to a composition, said composition comprising: a therapeutically effective amount of a GnRH antagonist in combination with a multi-block copolymer, wherein said polymer comprises polyethyleleglycol(PEG)-PLGA-PEG, poly(3-hydroxybutyrate), PCL, PLG, PLA, or a combination thereof.

In another aspect, the invention relates to a composition, said composition comprising: a therapeutically effective amount of a GnRH antagonist in combination with a multi-block copolymer, wherein said multi-block copolymer comprises randomly or non alternatingly arranged hydrolysable segments, wherein each segment comprises pre-polymer A or pre-polymer B, and wherein said segments are operably linked to each other by a multifunctional chain extender. In an exemplary embodiment, said multi-block copolymer is a phase separated multiblock copolymer, comprising: one or more segments of a linear soft biodegradable pre-polymer A having a glass transition temperature (T_{g}) lower than 37°C.; and one or more segments of a linear hard biodegradable pre-polymer B having a melting point temperature (Tₘ) of 40-100° C.

In another aspect, the invention relates to the use of salt bridges or cyclization of the active agent either as a primary drug delivery technique or in combination with another drug delivery vehicle using compounds that include, but are not limited to, lanthionine, dicarba, hydrazine, or lactam bridges.

In another aspect, the invention relates to the use of micronization or stabilizing adjuvants for a long term delivery of a GnRH antagonist.

In another aspect, the invention relates to the use of a solid-in-oil-in-water (S/O/W), a water-in-oil-in water (W/O/W), or a water-oil (W/O) production method for long term delivery of a GnRH antagonist.

In an exemplary embodiment, the composition is capable of achieving a therapeutic effect within, for example, 24 hrs and maintains therapeutic effect for at least 90 days for >95% percent of treated patients. In a particular embodiment, the composition is in the form of a hydrogel. In another particular embodiment, the composition is in the form of microspheres.

The composition of the invention can administered using a suitable method. In one aspect, the composition of the invention is an injectable composition, which is administered with one injection or two injections administered at the same time using, for example, a 21 gauge needle or smaller, with a total injection volume, for example, less than 4mL. Injections may be subcutaneous or intramuscular.

In another aspect, the invention relates to a composition for a long-term release of cetrorelix, the composition comprising a biodegradable polymer, a solvent, and a therapeutically effective amount of cetrorelix.

In another aspect, the invention relates to a method for extending the release of cetrorelix in a subject for a period ranging from about 1 month to about 6 months, the method comprising administering to said subject a composition comprising cetrorelix and a polymer, said polymer comprising or poly-lactic co-glycolic acid (PLGA) in a lactide:glycolide molar ratio between 50:50 and 100:0, wherein cetrorelix is present in an amount of 5%-90% of the mass of said composition, and said polymer is present in an amount of 10%-50% of the mass of said composition.

In another aspect, the invention relates to a method of maintaining a therapeutic level of cetrorelix in a subject for a period ranging from about 1 month to about 6 months, the method comprising administering to said subject a composition comprising cetrorelix and a polymer, said polymer comprising or poly-lactic co-glycolic acid (PLGA) in a lactide:glycolide molar ratio between 50:50 and 100:0, wherein cetrorelix is present in an amount of 5%-90% of the mass of said composition, and said polymer is present in an amount of 10%-50% of the mass of said composition.

In another aspect, the composition of the invention allows for consistent release of the active agent from the drug delivery vehicle with no more than 25% variation plus an encapsulation efficiency of over 70%. In yet another aspect, the composition of the invention allows Releases the active agent from the drug delivery vehicle with >85% intact over the entire duration of release.

Other features and advantages of the present invention will become apparent from the following detailed description examples and figures. It should be understood, however, that the detailed description and the specific examples while indicating preferred embodiments of the invention are given by way of illustration only, since various changes and modifications within the spirit and scope of the invention will become apparent to those skilled in the art from this detailed description.

### BRIEF DESCRIPTION OF THE DRAWINGS

**Figure1** shows *in vitro* release of cetrorelix (CRX) from microspheres composed of different polymers and carriers.
**Figure 2** shows *in vitro* release of cetrorelix from microspheres composed of different polymers loaded with cetrorelix in 35% Acetic acid/65% H₂O carrier.
**Figure 3** shows daily levels of cetrorelix release from microspheres composed of different polymers.
**Figure 4A** shows the morphology of cetrorelix coated 10CP10C20-D23 beads (12.7% cetrorelix in 65% Acetic Acid (HAc):25% water).
**Figure 4B** shows the morphology of cetrorelix coated 20CP15C50-D23 beads comprising 13.4% cetrorelix).
**Figure 5** shows cetrorelix plasma concentration following administration of cetrorelix-loaded PLGA microspheres to rats.
**Figure 6A** shows long term cetrorelix plasma concentration following administration of microspheres loaded with cetrorelix and salt formulations. to rats.
**Figure 6B** shows plasma concentration following administration of microspheres loaded with cetrorelix and salt formulations over first 24 hours following administration (*i.e.* burst).
**Figure 7A** shows comparative cetrorelix plasma concentrations for microspheres loaded with cetrorelix with and without salt.
**Figure 7B** shows comparative cetrorelix plasma concentrations for microspheres loaded with cetrorelix with and without salt over first 24 hours following administration.
**Figure 8** shows shows comparative cetrorelix plasma concentrations for microspheres loaded with cetrorelix with and without salt after dose normalization.
**Figure 9A** shows rat serum testosterone levels following administration of various cetrorelix microspheres formulations.
**Figure 9B** shows rat serum testosterone levels following administration of various cetrorelix microspheres formulations over first 24 hours following administration (*i.e.* burst).
**Figure 10** shows cumulative cetrorelix *in vitro* release from PLGA microspheres.
**Figure 11** shows cumulative cetrorelix *in vitro* release from RG502H/RG752H (30%PLGA)-salt microsphere formulations.
**Figure 12** shows cumulative cetrorelix *in vitro* release from RG752H (40% PLGA) salt microsphere formulations.
**Figure 13** shows cumulative cetrorelix *in vitro* release from RG502H/RG752H (40%PLGA)-salt microsphere formulations.

### DETAILED DESCRIPTION OF THE INVENTION

The invention relates to a controlled release composition comprising a Gonadotropin-releasing hormone (GnRH) antagonist in combination with one or more polymers and/or salts.

The composition may include any suitable a GnRH antagonist, known to one of skilled in the art. GnRH is also known as follicle-stimulating hormone-releasing hormone (FSH-RH), luteinizing hormone-releasing hormone (LHRH), gonadoliberin, and by various other names, known to one of skilled in the art.

In a particular embodiment, the GnRH antagonist is cetrorelix, abarelix, degarelix, ganirelix, ozarelix, taverelix, antarelix, or iturelix.

In one aspect, provided herein is a composition, said composition comprising: a therapeutically effective amount of a GnRH antagonist in combination with a polymer, wherein said polymer is poly(glycolide) (PLG), poly(lactide) (PLA), or poly-lactic co-glycolic acid (PLGA), wherein said composition is capable of releasing said GnRH antagonist for a long term (e.g., more than 90 days).

In another aspect, the PLGA polymers in the compositions of the present invention may have lactide:glycolide weight ratio ranging from about 50:50 to about 100:0. In particular embodiments, the lactide to glycolide ratio is about, 50:50, 55:45, 60:40, 65:35, 70:30, 75:25, 80:20, 85:15, 90:10, or 95:5.

In a further aspect, the PLGA polymers the compositions of the present invention may comprise a mixture of two or more PLGA polymers each having a different glycolide and lactide fractions. For example, the mixture may include a first PLGA polymer having equal amount of glycolide and lactide (RG502H) and a second PLGA polymer having 25% glycolide and 75% lactide (RG752H). The proportions of the first PLGA polymer and the second PLGA polymer may vary, for example the ratio of RG502H to RG752H can range from about 100:0 to about 0:100.

In another aspect, provided herein is a composition, said composition comprising: a therapeutically effective amount of a GnRH antagonist in combination with a non-PLGA block polymer, wherein said composition is capable of releasing said GnRH antagonist for a long term. Non-PLGA polymers are well known in the art. Examples of a non-PLGA block polymer include, for example, but not limited to, polyethyleneglycol (PEG), PLG, PLA, polybutylene terephthalate (PBT), poly(epsilon-caprolactone) (PCL), dioxanone, butanediisocyanate, butanediol polyoxyetylene, polypropylene, polyoxypropylene, polystyrene, poly methyl methacylate, or a block copolymer which additionally incorporates one more novel amiphilic, hydrophilic, or hydrophobic component. In another aspect, a non-PLGA block polymer comprises a blend of two or more polymer types capable of releasing therapeutically effective amount of GnRH antagonists.

In one aspect, the composition is a flowable composition capable of forming an *in situ* implant in a subject. In one example, the composition includes a biodegradable thermoplastic polymer, a biocompatible solvent; and a GnRH antagonist.

In another aspect, the invention relates to a flowable composition, the composition comprising: (a) a biodegradable thermoplastic polyester that is at least substantially insoluble in aqueous medium or body fluid; (b) a biocompatible polar aprotic solvent, wherein the biocompatible polar aprotic solvent is miscible to dispersible in aqueous medium or body fluid; and (c) a therapeutically effective amount of cetrorelix.

The biodegradable thermoplastic polymer can be substantially insoluble in aqueous medium or body fluid. Biodegradable thermoplastic polymers are well known in the art and fully described in U.S. Patents 6,565,874; 5,324,519; 4,938,763; 5,702,716; 5,744,153; and 5,990,194, which are incorporated by reference herein in their entirety. In one embodiment, biodegradable thermoplastic polymer is a polyester, for example, but not limited to, a polylactide, a polyglycolide, a polycaprolactone, a copolymer thereof, a terpolymer thereof, or any combination thereof.

The type, amount, and molecular weight, of biodegradable thermoplastic polymer present in the composition may depend upon one or more desired properties of the controlled release implant.

Examples of types of biodegradable thermoplastic polyesters are well known in the art and fully described in U.S. Patent 6,565,874, which is incorporated by reference herein in its entirety. In a particular embodiment, the suitable biodegradable thermoplastic polyester is 50:50 poly (DL-lactide-co-glycolide) having a carboxy terminal group or is 75:25 poly (DL-lactide-co-glycolide) with a carboxy terminal group that is protected. Other suitable copolymers, known to one of skilled in the art, can also be used.

The amount of biodegradable thermoplastic polymer, in the composition, can be any suitable amount, known to one of skilled in the art. The amount, in the composition, may range from about 10 wt. % to about 80 wt. %; from about 20 wt. % to about 60 wt. %; from about 25 wt. % to about 55 wt. %; from about 30 wt. % to about 50 wt. %; or from about 35 wt. % to about 45 wt. %. In a particular embodiment, the amount is approximately 10, 20, 25, 30, 35, 40, 45, 50, 55, 60, 65, 70, or 80 wt. %.

The molecular weight of biodegradable thermoplastic polymer, in the composition, can be any suitable molecular weight, known to one of skilled in the art. The molecular weight may range from about 10,000 to about 50,000; from about 15,000 to about 45,000; from about 20,000 to about 40,000; or from about 20,000 to about 30,000. In a particular embodiment, the molecular weight is approximately 10,000, 15,000, 20,000, 25,000, 30,000, 35,000, 40,000, 45,000, or 50,000.

Preferably, the biodegradable thermoplastic polyester has an average molecular weight ranging from about 23,000 to about 45,000 or from about 15,000 to about 24,000.

The biocompatible solvent can be a biocompatible polar aprotic solvent. In one embodiment, the solvent is miscible to dispersible in aqueous medium or body fluid. Suitable polar aprotic solvents are well known in the art and fully described in, for example., in Aldrich Handbook of Fine Chemicals and Laboratory Equipment, Milwaukee, Wis. (2000) and U.S. Patents 6,565,875, 5,324,519; 4,938,763; 5,702,716; 5,744,153; and 5,990,194, which are incorporated by reference herein in their entirety.

In one aspect, the solvent of the invention is capable of diffusing into body fluid so that the flowable composition coagulates or solidifies. In another aspect, the solvent of the invention is biodegradable. In yet another aspect, the solvent of the invention is non-toxic. As set forth in U.S. Patent 6,565,875, examples of suitable polar aprotic solvents include polar aprotic solvents having an amide group, an ester group, a carbonate group, a ketone, an ether, a sulfonyl group, or a combination thereof.

In one embodiment, the polar aprotic solvent is N-methyl-2-pyrrolidone, 2-pyrrolidone, N, N-dimethylformamide, N, N-dimethylacetamide, dimethyl sulfoxide, propylene carbonate, caprolactam, triacetin, or any combination thereof. In another embodiment, the polar aprotic solvent is N-methyl-2-pyrrolidone.

As set forth in U.S. Patent 6,565,875, the polar aprotic solvent can be present in any suitable amount. The type and amount of biocompatible polar aprotic solvent present in the composition may depend upon the desired properties of the controlled release implant.

In a particular embodiment, the type and amount of biocompatible polar aprotic solvent can influence the length of time in which the GnRH antagonist is released from the controlled release implant.

In another aspect, the invention relates to a method of preparing a flowable composition, the method comprising: mixing a biodegradable thermoplastic polymer, a biocompatible solvent; and a GnRH antagonist. The mixing may be performed for a sufficient period of time effective to form the flowable composition for use as a controlled release implant.

In yet another aspect, the invention relates to an implant formed in situ by the process of injecting the composition of the invention to a subject; allowing the solvent, in said composition, to dissipate to produce a solid biodegradable implant.

In yet another aspect, the invention relates to a method of forming an implant in situ in a subject, the method comprising the steps of: injecting the composition of the invention to a subject; allowing the solvent, in said composition, to dissipate to produce a solid biodegradable implant.

In one example, the flowable composition of the invention comprises a flowable delivery system such as an Atrigel^{®} system comprising a copolymer, a water soluble organic solvent, and a bioactive agent, for example, a GnRH antagonist.

In yet another aspect, the invention relates to a method for treating a disease associated with GnRH, the method comprising administering a therapeutically effective amount of the composition of the invention.

In a further aspect, the invention relates to a method of extending release of a pharmaceutical agent (e.g. cetrorelix) in a subject for a period ranging from about 1 month to about 6 months, the method comprising administering to said subject a composition of the invention (e.g. microspheres). In another aspect, the invention relates to a method of extending the release of a pharmaceutical agent (*e.g.* cetrorelix) in a subject for a period of at least 90 days, the method comprising administering to said subject a composition of the invention (e.g. microspheres).

In a yet further aspect, the invention relates to a method of maintaining an effective level of a therapeutic agent (e.g. cetrorelix) in a subject for a period ranging from about 1 month to about 6 months, the method comprising administering to said subject a composition of the invention (e.g. microspheres). In another aspect, the invention relates to a method of maintaining an effective level of a therapeutic agent (e.g. cetrorelix) in a subject for a period at least 90 days, the method comprising administering to said subject a composition of the invention (e.g. microspheres).

The invention also relates to a kit, wherein the kit comprising: the composition of the invention.

The invention relates to a controlled release composition comprising a Gonadotropin-releasing hormone (GnRH) antagonist (e.g., cetrorelix) loaded in a multi-block copolymer.

In one aspect, the inventor relates to a multi-block copolymer composition having a Gonadotropin-releasing hormone (GnRH) antagonist (e.g., cetrorelix) as a bioactive agent. The multi-block copolymer compositions are well known and fully described in U.S. Patents 8,481,651; 8,674,032; 8,674,033; and 9,364,442 and U.S. Patent Application Publications 2013/0209568; 2013/0273284; and 2014/0199385, and PCT International Patent Application Publications WO2005068533; WO2004007588; WO2012005594; and WO2013015685, all of which are incorporated by reference herein in their entirety.

The multi-block copolymer comprises one or more hydrolysable segments. In one embodiment, the multi-block copolymer comprises one or more randomly arranged hydrolysable segments. In another embodiment, the multi-block copolymer comprises one or more non-randomly arranged hydrolysable segments. In yet another embodiment, the multi-block copolymer comprises one or more alternatingly arranged hydrolysable segments. In yet another embodiment, the multi-block copolymer comprises one or more non-alternatingly arranged hydrolysable segments.

In some embodiments, the segments can be randomly and non-alternatingly connected to each other by multi-functional chain extenders.

In one example, the multi-block copolymer is amorphous at human body conditions.

In an exemplary embodiment, the multi-block copolymer has a glass transition temperature below body temperature at human body conditions.

In another aspect, the multi-block copolymer includes pre-polymer A, pre-polymer B, or a combination thereof. In one embodiment, pre-polymers A and B are composed of different monomers. In another embodiment, pre-polymers A and B are composed of the same monomers but in a different amount. In yet another embodiment, the pre-polymers are composed of the same monomers but with a different initiator in order to obtain the multi-block copolymers of the present invention.

Pre-polymers A and B are selected in such a way that the segments would exhibit significantly different properties, for example, but not limited to thermal, degradation and hydrophilic properties.

The pre-polymers A or B may comprise a hydrolysable polyester, poly ether ester, polycarbonate, polyester carbonate, polyanhydride or copolymers thereof, derived from cyclic monomers such as lactide (L, D or L/D), glycolide, ε-caprolactone, δ-valerolactone, trimethylene carbonate, tetramethylene carbonate, 1,5-dioxepane-2-one, 1,4-dioxane-2-one (para-dioxanone) or cyclic anhydrides (oxepane-2,7-dione).

In one embodiment, pre-polymer includes ester. In another embodiment, pre-polymer includes carbonate. In yet another embodiment, pre-polymer includes an anhydride linkage. In some embodiments, pre-polymer optionally comprises a polyether group. In an exemplary embodiment, polyether is present as an additional pre-polymer.

In one example, pre-polymer comprises a reaction product of an ester forming monomer selected from the group consisting of diols, dicarboxylic acids and hydroxycarboxylic acids.

In another example, pre-polymer comprises reaction products of at least one suitable cyclic monomer with at least one non-cyclic initiator selected from the group consisting of diols, dicarboxylic acids and hydroxycarboxylic acids.

Examples of cyclic monomer include, for example, but not limited to, glycolide, lactide (L, D or DL), ε-caprolactone, δ-valerolactone, trimethylene carbonate, tetramethylene carbonate, 1,4-dioxane-2-one (para-dioxanone), 1,5-dioxepane-2-one and cyclic anhydrides.

In some embodiments, pre-polymer comprises at least two different cyclic monomers. In one example, pre-polymer comprises glycolide and ε-caprolactone in a 1:1 weight ratio. In another example, pre-polymer comprises glycolide and lactide in a 1:1 weight ratio.

Examples of non-cyclic initiator include, for example, but not limited to, succinic acid, glutaric acid, adipic acid, sebacic acid, lactic acid, glycolic acid, hydroxybutyric acid, ethylene glycol, diethylene glycol, 1,4-butanediol and 1,6-hexanediol.

Examples of polyether groups include, for example, but not limited to, PEG (polyethylene glycol), PEG-PPG (polypropylene glycol), PTMG (polytetramethylene ether glycol) and combinations thereof. In a particular embodiment, the polyether group is PEG. PEG can be an initiator for ring-opening polymerization. PEG with any suitable molecular weight can be used, for example, a molecular weight between 150-4000. In one embodiment, each of pre-polymers A and B has a number average molecular weight between 300 and 30000.

In a particular embodiment, the composition comprises a polyethylene glycol (PEG). Any suitable PEG known to one of skilled in the art can be used. In an exemplary embodiment, PEG is polyethylene glycol 200, polyethylene glycol 300, or methoxy polyethylene glycol 350.

The chain-extender of the invention can be any suitable multifunctional chain extender, known to one of skilled in the art. In one embodiment, the pre-polymers are linked by the di-functional chain-extender. Examples of di-functional chain-extender include, for example, but not limited to, a diisocyanate chain-extender, a diacid and a diol compound.

The amount of pre-polymer, in the composition, can be any suitable amount, known to one of skilled in the art. The amount, in the composition, may be of about 10-90 wt. %.

The methods for synthesis of pre-polymers and multi-block copolymer compositions are well known and fully described in U.S. Patents 8,481,651; 8,674,032; 8,674,033; and 9,364,442 and U.S. Patent Application Publications 2013/0209568; 2013/0273284; and 2014/0199385, and PCT International Patent Application Publications WO2005068533; WO2004007588; WO2012005594; and WO2013015685, all of which are incorporated by reference herein in their entirety.

The intrinsic viscosity also may vary depending on one or more desired properties. In some embodiment, the intrinsic viscosity is larger than about 0.1 dl/g and less than about 6 dl/g. In one embodiment, the intrinsic viscosity lies between about 0.2-4 dl/g, more preferably between 0.4-2 dl/g.

In another aspect, the invention relates to phase separated multi block copolymers. The term "phase-separated," as used herein, may refer to a system, for example, a copolymer having two or more different pre-polymers, of which at least two are incompatible with each other at temperatures of 40° C or below (when kept at body conditions). As a result, the pre-polymers do not form a homogeneous mixture when combined as a physical mixture or chemical mixture.

The phase separated multi block copolymers are well known in the art and fully described in U.S. Patents 9,364,442 and 8,674,033, and PCT International Patent Application Publications WO2012005594 and, WO2004007588 which are incorporated by reference herein in their entirety. The phase-separated quality of the copolymers of the present invention is reflected in the profile of the glass transition temperature (Tg), melting temperature (Tm), or a combination thereof. For example, the phase-separated copolymers are characterized by at least two phase transitions, each of which is related to (but not necessarily identical to) the corresponding Tg or Tm values of the prepolymers which are comprised in the copolymer. In an exemplary embodiment, the multi-block copolymer is a phase separated multiblock copolymer, comprising: one or more segments of a pre-polymer A (e.g, a linear soft biodegradable pre-polymer A) having a glass transition temperature (T_{g}) lower than 37°C.; and one or more segments of a pre-polymer B (e.g., a linear hard biodegradable pre-polymer B) having a melting point temperature (Tₘ) ranging from about 40° C to about 100° C.

In another aspect, the invention relates to a composition, said composition comprising: a therapeutically effective amount of a cetrorelix in combination with a multi-block copolymer, wherein said multi-block copolymer comprises randomly or non alternatingly arranged hydrolysable segments, wherein each segment comprises pre-polymer A or pre-polymer B, and wherein said segments are operably linked to each other by a multifunctional chain extender. In an exemplary embodiment, said multi-block copolymer is a phase separated multiblock copolymer, comprising: one or more segments of a linear soft biodegradable pre-polymer A having a glass transition temperature (T_{g}) lower than 37°C.; and one or more segments of a linear hard biodegradable pre-polymer B having a melting point temperature (Tₘ) of 40-100° C.

The multi-block copolymer compositions may be in any suitable form, for example, in the form of implant, microspheres, microrods, microparticles, injectable gel formulation, coatings or membranes or devices, or any other form known in the art.

In another aspect, the invention relates to the use of salt bridges or cyclization of the active agent either as a primary drug delivery technique or in combination with another drug delivery vehicle using compounds that include, but are not limited to, lanthionine, dicarba, hydrazine, or lactam bridges. The formation of salt bridges for linking through non-covalent bonds are well known in the art and fully described in PCT patent application publications WO2009/155257 and WO 2012/163519, which are incorporated by reference herein in their entirety.

In another aspect, the invention relates to the use of micronization or stabilizing adjuvants for a long term delivery of a GnRH antagonist. Micronization techniques are well known in the art and fully described, for example, in PCT international Patent Application Publication WO2011/034514 and U.S. Patent Application Publication US2014/0219954, all of which are incorporated by reference herein in their entirety. Stabilizing adjuvants are also well known in the art and fully described, for example, in U.S. Patent 7,611,709, which is incorporated by reference herein in its entirety.

In another aspect, the invention relates to the use of a solid-in-oil-in-water (S/O/W), a water-in-oil-in water (W/O/W), or a water-oil (W/O) production method for long term delivery of a GnRH antagonist. These methods are well known in the art and fully described, for example, in PCT international Patent Application Publications WO2015/145353; WO2003/099262; and WO2007/129926 and U.S. Patent Application Publications US2002/0055461; US2008/0268004; and US2010/0019403, which are incorporated by reference herein in their entirety.

In an exemplary embodiment, the composition is capable of achieving a therapeutic effect, for example, within 24 hrs and maintains therapeutic effect for at least 90 days in, for example, >95% percent of treated patients. In a particular embodiment, the composition is in the form of a hydrogel. In another particular embodiment, the composition is in the form of microspheres.

Microspheres for sustained release of therapeutically active agents and methods of their preparations are well known in the art (*see e.g.* U.S. Patent Nos. 6,458,387 and 9,381,159 incorporated by reference herein in their entirety). The microspheres typically comprise a matrix formed of biodegradable polymer. In some embodiments, the inner matrix diffuses through the outer surface under appropriate conditions. In some embodiments, the outer surface not only allows aqueous fluids to enter the microsphere, but also allows solubilized drug and polymer to exit the microsphere. The microspheres can be made to release drug and polymer from the interior of the microsphere when placed in an appropriate aqueous medium, such as body fluids or a physiologically acceptable buffer under physiological conditions over a prolonged period of time, thereby providing sustained release of a drug. In one embodiment, the microspheres can be made to release a drug without an initial burst or rapid drug release.

The microspheres have a generally uniform size (substantially spherical) and shape, with each preparation having a narrow size distribution. Microspheres range in size from about 0.5 microns to about 100 microns, depending upon the fabrication conditions. The characteristics of the microspheres may be altered during preparation by manipulating the water soluble polymer concentration, reaction temperature, pH, concentration of therapeutic agent, crosslinking agent, and/or the length of time the macromolecule is exposed to the crosslinking agent and/or the energy source. In one example, microspheres are suitable for oral or parenteral administration; mucosal administration; ophthalmic administration; intravenous, subcutaneous, intra articular, or intramuscular injection; administration by inhalation; or topical administration.

The amount of polymer matrix, in the microsphere composition, can be any suitable amount, known to one of skilled in the art. The amount, in the microsphere composition, may range from about 10 wt. % to about 50 wt. %; from about 20 wt. % to about 40 wt. %; from about 25 wt. % to about 35 wt. %. In a particular embodiment, the amount is approximately 10, 20, 25, 30, 35, 40, 45, or 50 wt. %.

The amount of therapeutic molecule in the microsphere can range from about 1 wt. % to about 90 wt. %, from about 1 wt. % to about 40 wt. %, from about 3 wt. % to about 30 wt. %, from about 5 wt. %. to about 20 wt. %, from about 10 wt. %. to about 15 wt. %. In a particular embodiment, the amount is approximately 1, 3, 5, 7, 9 10, 15 20, 25, 30, 35, 40, 45, 50, 60, 70, 80, or 90 wt. %.

The composition of the invention can be administered using a suitable method. In one aspect, the composition of the invention is an injectable composition, which is administered with one injection or two injections administered at the same time using, for example, a 21G needle or smaller, with a total injection volume, for example, less than 4mL. Injections may be subcutaneous or intramuscular.

In another aspect, the composition of the invention allows for consistent release of the active agent from the drug delivery vehicle with no more than 25% variation plus an encapsulation efficiency of over 70%. In yet another aspect, the composition of the invention allows the releases the active agent from the drug delivery vehicle with >85% intact over the entire duration of release.

Effective doses of the compositions of the present invention, for treatment of conditions or diseases as described herein vary depending upon many different factors, including means of administration, target site, physiological state of the patient, whether the patient is human or an animal, other medications administered, and whether treatment is prophylactic or therapeutic. Usually, the patient is a human but non-human mammals including transgenic mammals can also be treated. Treatment dosages may be titrated using routine methods known to those of skill in the art to optimize safety and efficacy.

The pharmaceutical compositions of the invention may include a "therapeutically effective amount." A "therapeutically effective amount" refers to an amount effective, at dosages and for periods of time necessary, to achieve the desired therapeutic result. A therapeutically effective amount of a molecule may vary according to factors such as the disease state, age, sex, and weight of the individual, and the ability of the molecule to elicit a desired response in the individual. A therapeutically effective amount is also one in which any toxic or detrimental effects of the molecule are outweighed by the therapeutically beneficial effects.

The invention further provides methods for treating a disease or condition with gonadotropin-releasing hormone (GnRH) antagonist, thereby treating said disease in said subject.

The compositions of the invention described herein can be used to treat any GnRH associated disease or condition that could be treated by GnRH antagonist. Examples of treatments, for diseases or conditions treated by the compositions of the invention include, for example, but not limited to, suppression of testosterone production, FSH, and LH for the treatment of prostate cancer and benign prostatic hyperplasia, directly blocking GnRH receptors on prostate cells for treatment of prostate cancer and benign prostatic hyperplasia, controlled ovarian stimulation for assisted reproductive techniques, treatment of uterine myoma, suppression of ovarian function while undergoing chemotherapy, treatment of breast cancer, treatment of ovarian cancer, male contraception, and female contraception.

As used herein, the terms "treat" and "treatment" refer to therapeutic treatment, including prophylactic or preventative measures, wherein the object is to prevent or slow down (lessen) an undesired physiological change associated with a disease or condition. Beneficial or desired clinical results include, but are not limited to, alleviation of symptoms, diminishment of the extent of a disease or condition, stabilization of a disease or condition (*i.e.*, where the disease or condition does not worsen), delay or slowing of the progression of a disease or condition, amelioration or palliation of the disease or condition, and remission (whether partial or total) of the disease or condition, whether detectable or undetectable. "Treatment" can also mean prolonging survival as compared to expected survival if not receiving treatment. Those in need of treatment include those already with the disease or condition as well as those prone to having the disease or condition or those in which the disease or condition is to be prevented.

"Administration" to a subject is not limited to any particular delivery system and may include, without limitation, parenteral (including subcutaneous, intravenous, intramedullary, intraarticular, intramuscular, or intraperitoneal injection).

The composition of the invention may be administered parenterally (e.g., intravenous, subcutaneous, intraperitoneal, and intramuscular). Further, the composition of the invention may be administered by intravenous infusion or injection. The composition of the invention may be administered by intramuscular or subcutaneous injection. In some embodiments, the composition of the invention may be administered surgically. As used herein, a "composition" refers to any composition that contains a pharmaceutically effective amount of one or more active ingredients (e.g., a GnRH antagonist).

The methods of treatment described herein can be used to treat any suitable mammal, including primates, such as monkeys and humans, horses, cows, cats, dogs, rabbits, elk, deer and rodents such as rats and mice. In one embodiment, the mammal to be treated is human.

All patents and literature references cited in the present specification are hereby incorporated by reference in their entirety.

Preferred embodiments of the invention are compiled as clauses 1 to 68 hereinafter:
1. A long-term drug release composition comprising: a therapeutically effective amount of a GnRH antagonist in combination with a polymer, wherein said composition is capable of releasing said GnRH antagonist for a long term.
2. The composition of clause 1, wherein said composition is capable of releasing said GnRH antagonist for more than 90 days.
3. The composition of clause 1, wherein said composition is capable of achieving a therapeutic effect within 24 hrs and maintains therapeutic effect for at least 90 days.
4. The composition of clause 1, wherein said composition is in the form of a hydrogel.
5. The composition of clause 1, wherein said composition is a flowable composition.
6. The composition of clause 1, wherein said composition is in the form of a microsphere.
7. The composition of clause 1, wherein said composition is in the form of an implant.
8. The composition of clause 1, wherein said GnRH antagonist is cetrorelix, abarelix, degarelix, ganirelix, ozarelix, taverelix, antarelix, or iturelix.
9. The composition of clause 1, wherein said polymer is poly(glycolide) (PLG), poly (lactide) (PLA), or poly-lactic co-glycolic acid (PLGA).
10. The composition of clause 1, wherein said polymer is a non-PLGA polymer.
11. The composition of clause 10, wherein said non-PLGA polymer is poly ethyleneglycol (PEG), PLG, PLA, polybutylene terephthalate (PBT), poly(epsilon-caprolactone) (PCL), dioxanone, butanediisocyanate, butanediol, or a combination thereof.
12. A flowable composition, the composition comprising: (a) a biodegradable thermoplastic polyester that is substantially insoluble in aqueous medium or body fluid; (b) a biocompatible polar aprotic solvent, wherein the biocompatible polar aprotic solvent is miscible to dispersible in aqueous medium or body fluid; and (c) a therapeutically effective amount of a GnRH antagonist.
13. The composition of clause 12, wherein said flowable composition is capable of forming an implant *in situ,* after its administration into a subject.
14. The composition of clause 12, wherein said flowable composition is an injectable composition.
15. The composition of clause 12, wherein said flowable composition is injectable intramuscularly or subcutaneousy.
16. The composition of clause 12, wherein said biodegradable thermoplastic polymer is substantially insoluble in aqueous medium or body fluid.
17. The composition of clause 16, wherein the thermoplastic polyester is a polylactide, a polyglycolide, a polycaprolactone, a copolymer thereof, a terpolymer thereof, or any combination thereof.
18. The composition of clause 12, wherein the solvent is capable of diffusing into body fluid so that the flowable composition coagulates or solidifies.
19. The composition of clause 18, wherein the solvent is N-methyl-2-pyrrolidone, 2-pyrrolidone, N,N-dimethylformamide, dimethyl sulfoxide, propylene carbonate, caprolactam, triacetin, or any combination thereof.
20. The composition of clause 12, wherein said flowable composition comprises an Atrigel^{®} delivery system, said system comprising a copolymer, a water soluble organic solvent, and said GnRH antagonist.
21. A method of preparing a flowable composition of clause 12, the method comprising: mixing a biodegradable thermoplastic polymer, a biocompatible solvent; and a Gonadotropin-releasing hormone (GnRH) antagonist.
22. An implant formed *in situ* by the process of injecting the composition of clause 12 to a subject; allowing the solvent, in said composition, to dissipate to produce a solid biodegradable implant.
23. A method of forming an implant *in situ* in a subject, the method comprising the steps of: injecting the composition of clause 12 to a subject; allowing the solvent, in said composition, to dissipate to produce a solid biodegradable implant.
24. A composition for a long-term release of cetrorelix, the composition comprising a biodegradable polymer, a solvent, and a therapeutically effective amount of cetrorelix.
25. The composition of clause 24, wherein said a polymer is poly-lactic co-glycolic acid (PLGA).
26. The composition of clause 25, wherein said PLGA comprises equal parts lactide and glycolide.
27. The composition of clause 25, wherein said PLGA comprises 75% lactide and 25% glycolide.
28. The composition of clause 25, wherein said PLGA comprises equal parts of a first and a second polymer composition, wherein said first polymer composition comprises equal parts lactide and glycolide and said second polymer composition comprises75% lactide and 25% glycolide.
29. The composition of clause 25, wherein the polymer is present at the concentration ranging from about 10% to about 50% (w/w).
30. The composition of clause 29, wherein polymer is present at the concentration ranging from about 20% to about 40% (w/w).
31. The composition of clause 25, wherein said solvent comprises about 50% acetic acid and about 50% water.
32. The composition of clause 25, wherein said solvent comprises about 35% Acetic acid and about 65% water.
33. The composition of clause 24, wherein said solvent is a polar aprotic solvent.
34. The composition of clause 33, wherein said solvent is N-methyl-2-pyrrolidone.
35. The composition of clause 24 further comprising a salt.
36. The composition of clause 35, wherein said salt is Ca pamoate, Na oleate, or Ca Citrate.
37. The composition of clause 24, wherein said solvent is present at the concentration ranging from about 10% to about 30% (w/w).
38. The composition of clause 24, wherein cetrorelix is present at the concentration ranging from about 5% to about 90% (w/w).
39. The composition of clause 24, wherein said composition is capable of achieving a therapeutic effect within 24 hrs and maintains therapeutic effect for at least 90 days.
40. The composition of clause 24, wherein said composition is in the form of a hydrogel.
41. The composition of clause 24, wherein said composition is a flowable composition.
42. The composition of clause 24, wherein said composition is in the form of a microsphere.
43. The composition of clause 24, wherein said composition is in the form of an implant.
44. A method for extending the release cetrorelix in a subject for a period ranging from about 1 month to about 6 months, the method comprising administering to said subject a composition comprising cetrorelix and a polymer, said polymer comprising or poly-lactic co-glycolic acid (PLGA) in a lactide:glycolide molar ratio between 50:50 and 100:0, wherein cetrorelix is present in an amount of 5%-90% of the mass of said composition, and said polymer is present in an amount of 10%-50% of the mass of said composition.
45. The method of clause 44, wherein said composition is in the form of a microsphere.
46. The method of clause 44, wherein lactide:glycolide molar ratio between 50:50 and 75:25.
47. The method of clause 44, wherein said polymer is present in an amount of 20%-40% of the mass of said implant.
48. A method for maintaining a therapeutic level of cetrorelix in a subject for a period ranging from about 1 month to about 6 months, the method comprising administering to said subject a composition comprising cetrorelix and a polymer, said polymer comprising or poly-lactic co-glycolic acid (PLGA) in a lactide:glycolide molar ratio between 50:50 and 100:0, wherein cetrorelix is present in an amount of 5%-90% of the mass of said implant, and said polymer is present in an amount of 10%-50% of the mass of said implant.
49. The method of clause 48, wherein said composition is in the form of a microsphere.
50. The method of clause 48, wherein lactide:glycolide molar ratio between 50:50 and 75:25.
51. The method of clause 48, wherein said polymer is present in an amount of 20%-40% of the mass of said implant.A composition comprising: a therapeutically effective amount of a GnRH antagonist in combination with a multi-block copolymer, wherein said polymer comprises polyethyleleglycol(PEG)-PLGA-PEG, poly(3-hydroxybutyrate), PCL, PLG, PLA, or a combination thereof.
52. A composition comprising: a therapeutically effective amount of a GnRH antagonist in combination with a multi-block copolymer, wherein said multi-block copolymer comprises randomly or non alternatingly arranged hydrolysable segments, wherein each segment comprises pre-polymer A or pre-polymer B, and wherein said segments are operably linked to each other by a multifunctional chain extender.
53. The composition of clause 52, wherein the segments are randomly or non-alternatingly linked to each other by a multi-functional chain extender.
54. The composition of clause 52, wherein the multi-block copolymer is amorphous at human body conditions.
55. The composition of clause 52, wherein the multi-block copolymer has a glass transition temperature below body temperature at human body conditions.
56. The composition of clause 52, wherein the multi-block copolymer includes pre-polymer A, pre-polymer B, or a combination thereof.
57. The composition of clause 56, wherein said pre-polymers A and B are composed of different monomers.
58. The composition of clause 56, wherein said pre-polymers A and B are composed of the same monomers but in a different amount.
59. The composition of clause 56, wherein said pre-polymers are composed of the same monomers but with a different initiator in order to obtain the multi-block copolymers.
60. The composition of clause 56, wherein said pre-polymers A or B comprises a hydrolysable polyester, poly ether ester, polycarbonate, polyester carbonate, polyanhydride or copolymers thereof, derived from cyclic monomers such as lactide (L, D or L/D), glycolide, ε-caprolactone, δ-valerolactone, trimethylene carbonate, tetramethylene carbonate, 1,5-dioxepane-2-one, 1,4-dioxane-2-one (para-dioxanone) or cyclic anhydrides (oxepane-2,7-dione).
61. The composition of clause 60, wherein said cyclic monomer is glycolide, lactide (L, D or DL), ε-caprolactone, δ-valerolactone, trimethylene carbonate, tetramethylene carbonate, 1,4-dioxane-2-one (para-dioxanone), 1,5-dioxepane-2-one, or a cyclic anhydride.
62. The composition of clause 60, wherein said polyether is PEG (polyethylene glycol), PEG-PPG (polypropylene glycol), PTMG (polytetramethylene ether glycol) and combinations thereof.
63. The composition of clause 56, wherein said multi-block copolymer is a phase separated multiblock copolymer.
64. The composition of clause 63, wherein said phase separated multiblock copolymer comprises one or more segments of a linear soft biodegradable pre-polymer A having a glass transition temperature (T_{g}) lower than 37°C.; and one or more segments of a linear hard biodegradable pre-polymer B having a melting point temperature (Tₘ) of 40-100° C.
65. A method for treating a disease or condition associated with gonadotropin-releasing hormone (GnRH), the method comprising administering to a subject a composition of any of the above clauses, thereby treating said disease in said subject.
66. The method of clause 65, wherein said treatment is suppression of testosterone production, FSH, and LH for the treatment of prostate cancer and benign prostatic hyperplasia, directly blocking GnRH receptors on prostate cells for treatment of prostate cancer and benign prostatic hyperplasia, controlled ovarian stimulation for assisted reproductive techniques, treatment of uterine myoma, suppression of ovarian function while undergoing chemotherapy, treatment of breast cancer, treatment of ovarian cancer, male contraception, and female contraception.
67. A composition comprising cetrorelix and a polymer, said polymer comprising or poly-lactic co-glycolic acid (PLGA) in a lactide:glycolide molar ratio between 50:50 and 100:0, wherein cetrorelix is present in an amount of 5%-90% of the mass of said composition, and said polymer is present in an amount of 10%-50% of the mass of said composition, and wherein said composition is capable of extending the release of cetrorelix in a subject for a period ranging from about 1 month to about 6 months.
68. The composition of clause 67, wherein said composition maintains a therapeutic level of cetrorelix in a subject for a period ranging from about 1 month to about 6 months.

### EXAMPLES

### EXAMPLE 1

Poly(DL-lactide-co-glycolide) with 50:50 ratio of lactide to glycolide can be dissolved in a suitable solvent to prepare an Atrigel^{®} polymer solution. This solution can be filled into a syringe with a female luer lock fitting.

Each GnRH antagonist (ozarelix, degarelix, cetrorelix, or ganirelex) can be dissolved in water or other solvents and filled into a syringe with a male luer-lock fitting.

Prior to administration, the two syringes can be coupled and the contents can be mixed back and forth between the two syringes for multiple cycles. After thorough mixing, the formulation can be drawn back into the syringe with the male coupling.

Then, the two syringes can be separated and a needle (a 21G needle or smaller) can be attached. The contents of the syringe can then be subcutaneously injected into subjects. A total injection volume can be less than 4mL.

Serum can be collected and analyzed. The GnRH antagonist composition may achieve a therapeutic effect within 24 hrs and maintain therapeutic effect for at least 90 days in >95% percent of treated patients.

The composition may allow for consistent release of the active agent from the drug delivery vehicle with no more than 25% variation plus an encapsulation efficiency of over 70%. The composition may release the active agent from the drug delivery vehicle with >85% intact over the entire duration of release.

### EXAMPLE 2

A multi-block copolymer is provided. Each GnRH antagonist (ozarelix, degarelix, cetrorelix, or ganirelex) can be loaded into the multi-block copolymer. The formulation may be in the form of microspheres.

A syringe with a 21G needle or smaller can be used to inject the formulation. The formulation can be subcutaneously injected into subjects. A total injection volume can be less than 4mL.

Serum can be collected and analyzed. The GnRH antagonist composition may achieve a therapeutic effect within 24 hrs and maintain therapeutic effect for at least 90 days in >95% percent of treated patients.

The composition may allow for consistent release of the active agent from the drug delivery vehicle with no more than 25% variation plus an encapsulation efficiency of over 70%. The composition may release the active agent from the drug delivery vehicle with >85% intact over the entire duration of release.

### EXAMPLE 3

### Development of Cetrorelix Microspheres Formulations

Several formulations of microspheres using different polymers and internal water phase compositions were prepared for testing cetrorelix *in vitro* release (IVR). The tested formulations are summarized in Table 1

**Table 1 Initial Cetrorelix Formulations**

| **MSP Batch** | **Process** | **Polymer** | **Microsphere morphology** | **Microsphere size (D50) (µm)** | **Theoretical CRX loading (Wt.%)** | **CRX Loading measured by EAS (Wt.%)** | **EE (%)** |
|---|---|---|---|---|---|---|---|
| **AD17-008** | W1/O/W2 (W1= Acetic acid/H₂O 50/50) | 10CP10 C20-D23 | Spherical, monodispersed | 40 | 12.5 | 11 | 88.5 |
| **RP17-004** | W1/O/W2 (W1= Acetic acid/H₂O 35/65 pre-mix) | 10CP10 C20-D23 | Spherical, monodispersed | 73 | 14.3 | 13.8 | 96.5 |
| **RP17-006** | W1/O/W2 (W1= Acetic acid/H₂O 35/65 pre-mix) | 10LP10 L20-LL40 | Spherical, monodispersed | 71 | 14.0% | 14.8 | 105.4 |

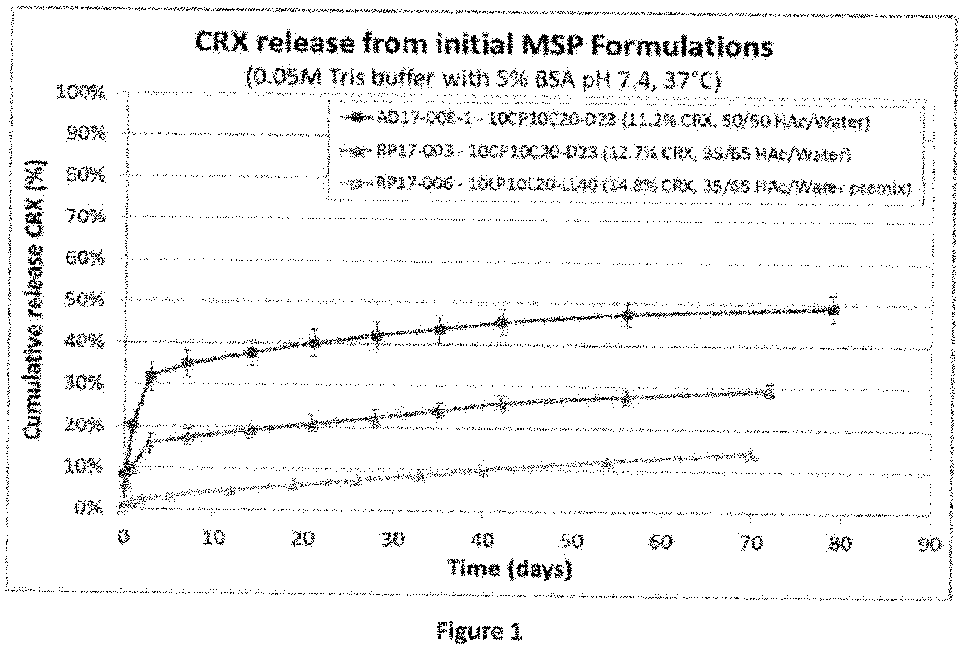

The *in vitro* release of cetrorelix was tested by incubating microsphere formulations listed in **Table 1** in 0.05 M Tris Buffer with 5% BSA, pH 7.4 at 37°C. The results show the release was slowest when premixed 35% Acetic acid/ 65% H₂O as internal water phase was used **(****Figure 1****).**

To further test cetrorelix IVR several formulations of cetrorelix-loaded microspheres using different polymers and 35% Acetic acid/ 65% H₂O as internal water phase were made **(Table 2).**

**Table 2 Cetrorelix Formulations manufactured with optimizing primary emulsification process (W1 = 36/65 Acetic acid/ Water mixture)**

| **MSP Batch** | **Process** | **Polymer** | **Microsphere morphology** | **MSP size (D50) (µm)** | **Theoretical CRX loading (Wt.%)** | **CRX Loading measured by EAS (Wt.%)** | **EE (%)** |
|---|---|---|---|---|---|---|---|
| **RP17-012** | W1/O/ W2 | 10CP10 C20-D23 | Spherical, monodispersed | 82 | 15.70% | 13.5 | 85.7 0% |
| **RP17-013** | W1/O/ W2 | 20CP15 C50-D23 | Spherical, monodispersed | 85 | 14.00% | 13.4 | 95.7 0% |
| **RP17-014** | W1/O/ W2 | 20LP10 C20-LL40 | Spherical, monodispersed | 70 | 13.90% | 13.6 | 97.8 0% |
| **RP17-015** | W1/O/ W2 | 20CP10C20 -LL40 | Spherical, monodispersed | 56 | 13.20% | 11.9 | 90.9 0% |
| **RP17-018** | W1/O/ W2 | 30CP15C50 -D23 | Spherical, monodispersed | 51 | 13.80% | 9.5 | 68.4 0% |

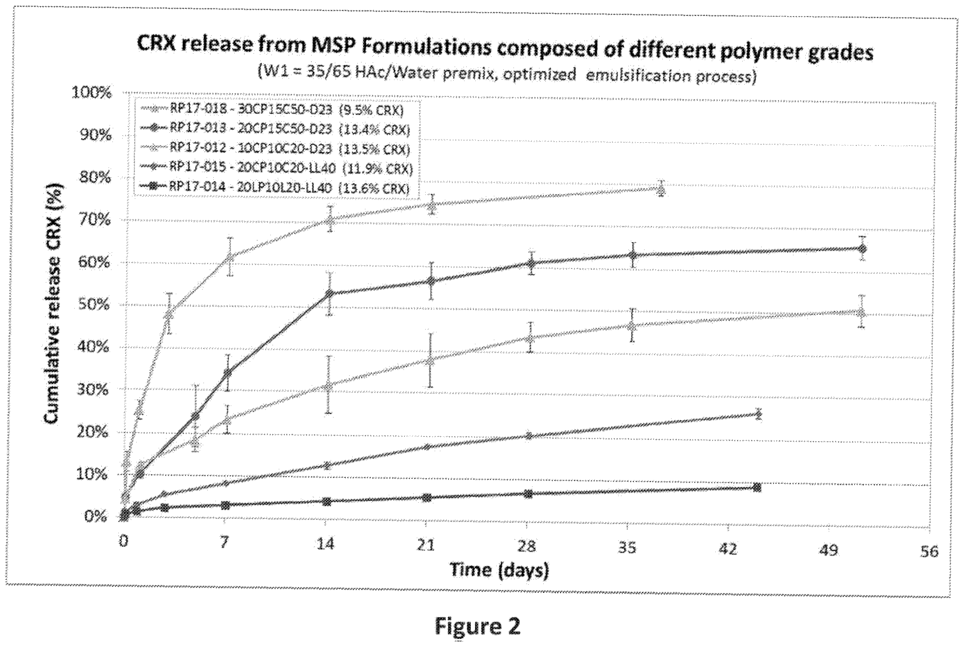

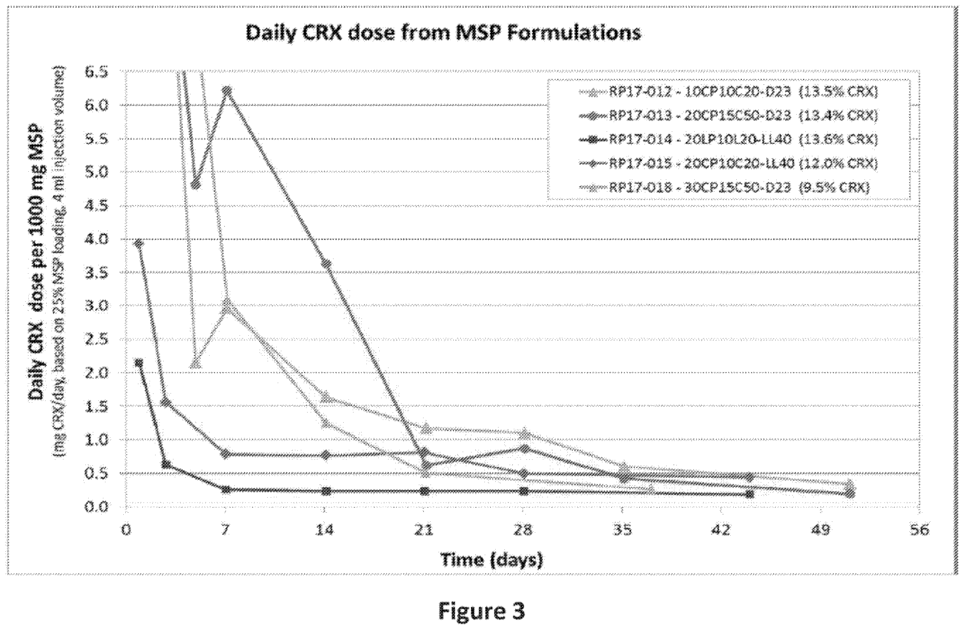

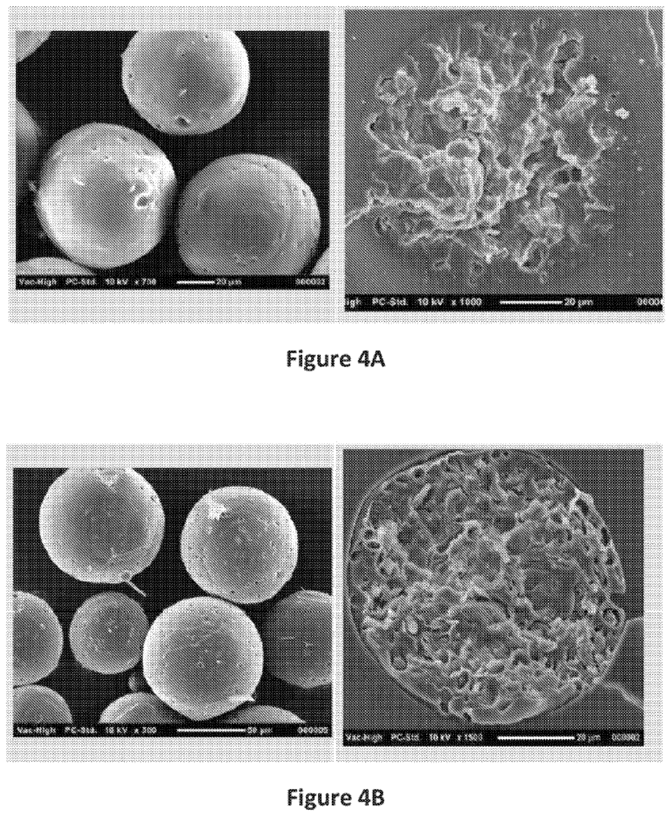

The *in vitro* release of cetrorelix was tested by incubating microsphere formulation listed in **Table 2** in 0.05 M Tris Buffer with 5% BSA, pH 7.4 at 37°C. The results show that the RP17-014 formulation using 20LP10C20-LL40 polymer had the slowest cetrorelix release rate, and, in addition shown linear release kinetics **(****Figure 2****),** while RP17-012 (10CP10C20-D23) and RP17-012 (10CP10C20-D23) microsphere formulations (depicted in electron micrographs in **Figure 4A** and **Figure 4B** respectively) provided the highest sustained daily dose of cetrorelix **(****Figure 3****).**

These results show that slow degrading L-Lactide based polymers with low swellability are suitable for use in cetrorelix-loaded microspheres and display linear cetrorelix release. In addition the microsphere manufacturing process achieves cetrorelix encapsulation of up to 15%.

### EXAMPLE 4

### Cetrorelix-Loaded Microspheres Pharmacokinetics in Rats

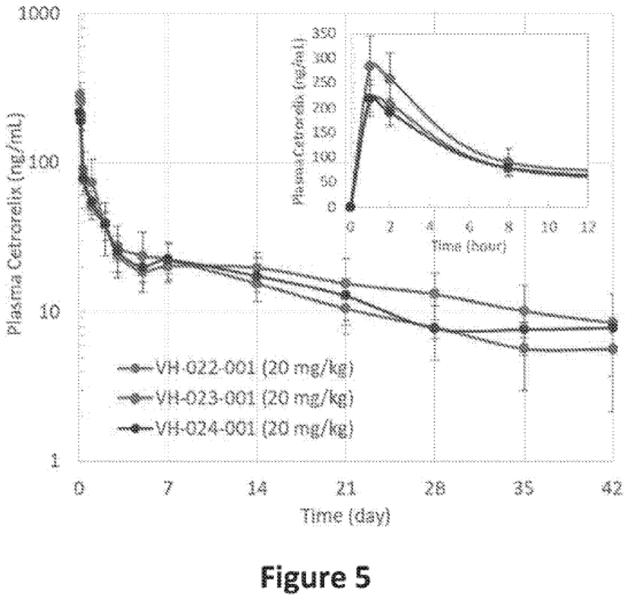

Several salt-free cetrorelix-loaded microsphere formulations having different polymer contents were used for PK Study **(Table3).** The preparations (<1 ml) were subcutaneously implanted into rats at a single 20 mg/kg dose and cetrorelix levels in plasma were monitored over 6 weeks. The results are summarized in **Table 4** and **Figure 5****.** All formulations showed detectable plasma cetrorelix ≥ 42 days. Cₘₐₓ/C_{ave,42day} varied from 12.1 to 17.6 and the percentage of day 1 release related to 42 days ranged from 12 to 17%. Importantly, the amount of polymer in the preparation did not seem to have any statistically significant effect on cetrorelix release.

**Table 3 Salt-free Cetrorelix-loaded microsphere formulations**

| **ID** | **Cetrorelix (mg)** | **RG502H (mg)** | **RG752H (mg)** | **NMP (mg)** | **Total (mg)** | **Theo. Cetrorelix Loading (%,w/w)** | **PLGA Solution (%,w/w)*** | **Total Solid (%,w/w)** |
|---|---|---|---|---|---|---|---|---|
| **VH-022-001** | 365.5 | 677.22 | 0.0 | 2688 | 3730.4 | 9.8 | 20.1% | 28.0 |
| **VH-023-001** | 291.3 | 408.28 | 405.7 | 1885 | 2990.5 | 9.7 | 30.2% | 37.0 |
| **VH-024-001** | 291.5 | 0.0 | 1084.6 | 1618 | 2993.6 | 9.7 | 40.1% | 46.0 |

| | | | | | | | | |
|---|---|---|---|---|---|---|---|---|
| ^{∗} PLGA (%,w/w) = PLGA wt ÷ (PLGA wt + Total Solvent wt) | | | | | | | | |

**Table 4 Salt-free Cetrorelix-loaded microspheres Rat pharmacokinetics results**

| **ID** | **Cₘₐₓ (ng/mL)** | **Tₘₐₓ (hr)** | **AUC_{0-42day} (ng/mL ∗ hr)** | **Cₐᵥₑ, 42day (ng/mL)** | **Cₘₐₓ/ Cₐᵥₑ, 42day** | **AUC₀₋₂₄ₕᵣ (ng/mL ∗ hr)** | **AUC₀₋₂₄ₕᵣ /AUC_{0- 42day}** |
|---|---|---|---|---|---|---|---|
| **VH-022-001** | 283.7 | 1 | 16272.7 | 16.1 | 17.6 | 2761.9 | 17.0 % |
| **VH-023-001** | 218.7 | 1 | 18270.5 | 18.1 | 12.1 | 2206.9 | 12.1 % |
| **VH-024-001** | 218.7 | 1 | 16406.1 | 16.3 | 13.4 | 2191.0 | 13.4 % |

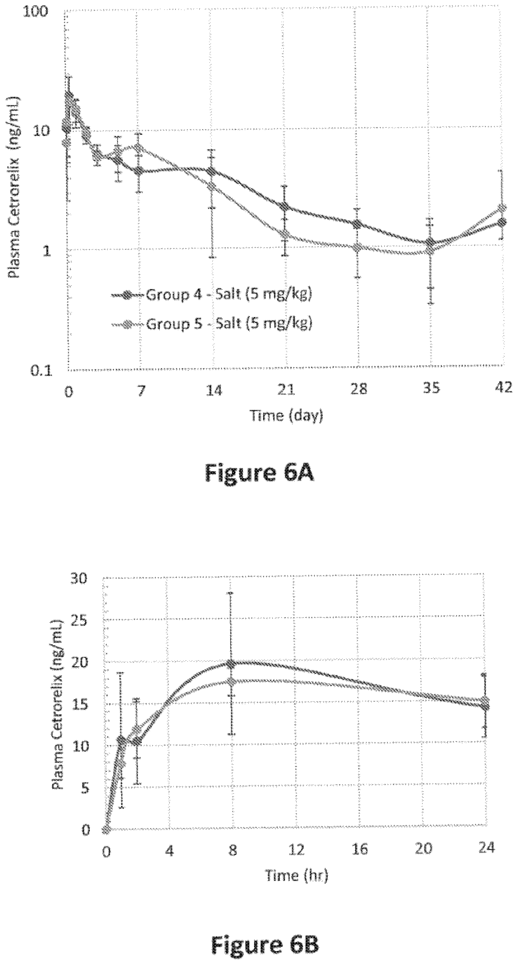

In addition, pharmacokinetics of cetrorelix microspheres formulations containing either Ca Pamoate or Na Oleate salt were tested in rats through subcutaneously implanting a single 5 mg/kg microspheres dose in a vehicle solution (20 mM K-Phos Buffer, 2.5% Mannitol, 3.5% CMC, 0.1% PS80) and monitoring cetrorelix levels in plasma over 6 weeks. The results are summarized in **Table 5** and **Figures 6A** and **6B****.** All formulations showed detectable plasma cetrorelix ≥ 42 days. Cₘₐₓ/C_{ave,42day} varied from 2.4 to 3.1 and the percentage of day 1 release related to 42 days about 11%.

**Table 5 Salt-containing Cetrorelix formulations Rat pharmacokinetics results.**

| **ID** | **Cₘₐₓ (ng/mL)** | **Tₘₐₓ (hr)** | **AUC_{0-42day} (ng/mL ∗ hr)** | **C_{ave, 42day} (ng/mL)** | **Cₘₐₓ/ C_{ave, 42day}** | **AUC_{0- 24hr} (ng/mL ∗ hr)** | **AUC_{0- 24hr}/ AUC_{0- 42day}** |
|---|---|---|---|---|---|---|---|
| **Group 4** - **Salt Ca Pamoate** | 19.6 | 8 | 3444.0 | 3.4 | 3.1 | 376.9 | 10.9% |
| **Group 5** - **Salt Na Oleate** | 17.5 | 8 | 3331.9 | 3.3 | 2.4 | 361.1 | 10.8% |

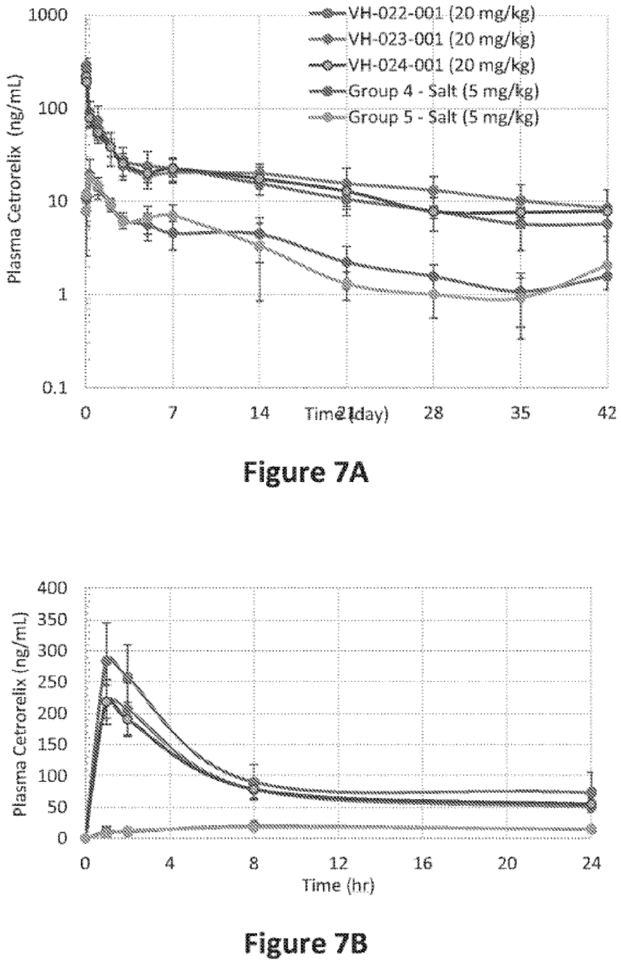

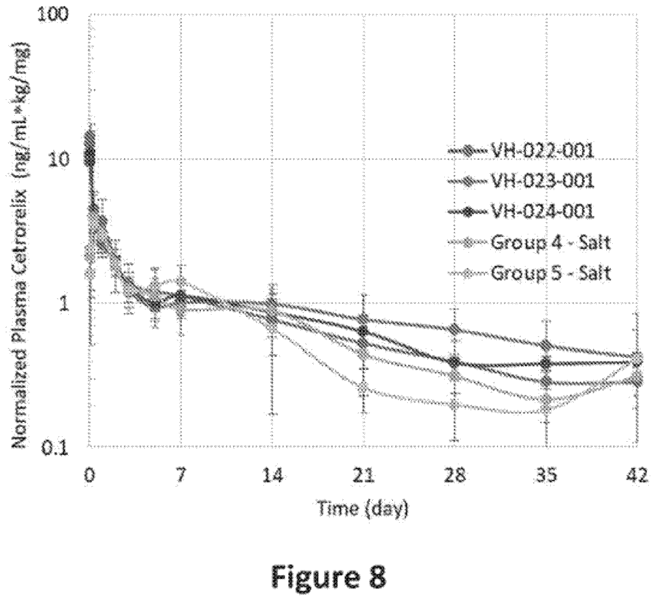

There was no principal difference between pharmacokinetics of cetrorelix microspheres formulations containing Ca Pamoate and those containing Na Oleate both in the initial 24 hour burst release **(****Figure 6B****)** and over the long term **(****Figure 6A****).** Moreover, while salt-containing cetrorelix microspheres resulted in lower plasma cetrorelix compared to the salt free formulations in the long term **(****Figure 7A****),** this difference could be attributable to the different dose **(****Figure 8****).** However, dose difference did not account for the far greater peak concentration observed at the initial burst release phase when salt free formulations were used. Indeed, addition of salt all but eliminated the sharp peak observed in its absence **(****Figure 7b****)**

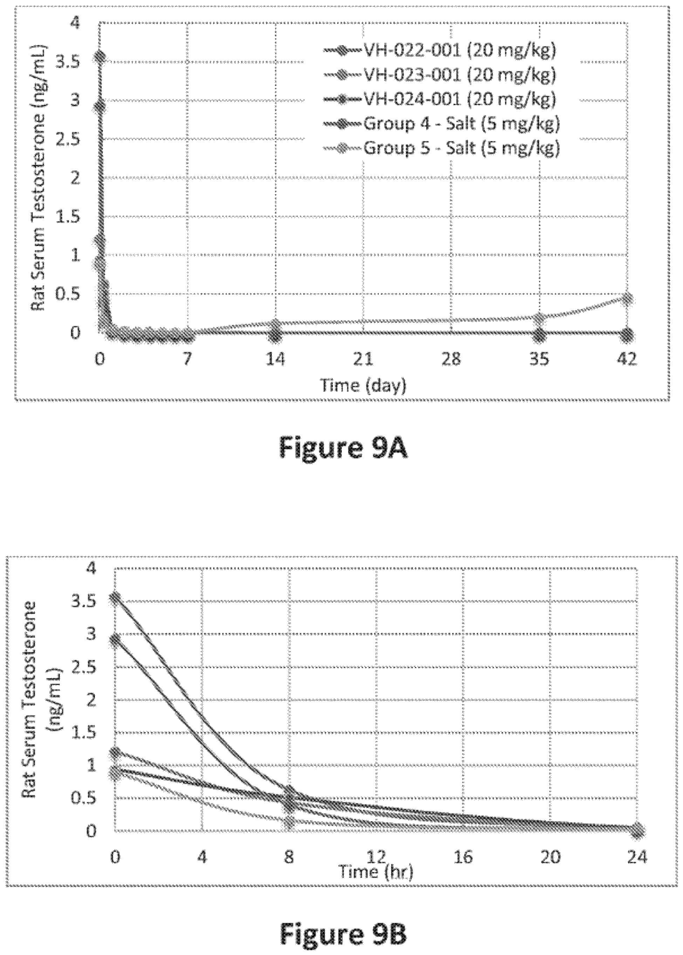

The downstream physiological effects of cetrorelix microspheres administration were assessed through monitoring testosterone levels in rats. All the formulations caused notable drop in serum testosterone levels after 24 hours **(****Figure 9B****).** These low levels were maintained for the entire 6 weeks monitoring period in all rats except those treated with Na Oleate- containing microspheres **(****Figure 9A****).**

The total amount of cetrorelix released was assessed using the method of Schwahn et al., Drug Metabolism & Disposition, Vol. 28, No. 1, p10, assuming rat weight ranging from 250 to 400 g, 10 mg/kg dose, and AUC (ng/mL ^{∗}hr) = 618.1.

Based on these assumptions, AUC for 20 mg/kg Dose (ng/mL ^{∗}hr) was assumed to be 123,620 and AUC for 5 mg/kg Dose (ng/mL ^{∗}hr) was assumed to be 30,905. The calculations based on the above assumptions results in estimated percentage of cetrorelix released (up to 42 days) ranging from 11 to 15 % of the amount initially present in the microspheres. (See **Table 6)**

**Table 6 Summary of pharmacokinetic data for Cetrorelix microspheres formulation**

| **ID** | **PLGA or Salt** | **Dose (mg/kg)** | **Cₘₐₓ (ng/mL)** | **Tₘₐₓ (hr)** | **AUC_{0-42day} (ng/mL ∗ hr)** | **Est. % Cetrorelix Released (up to 42 days)** |
|---|---|---|---|---|---|---|
| **VH-022-001** | RG502H, 20% | 20 | 283.7 | 1 | 16272.7 | 13.2 |
| **VH-023-001** | RG502H/RG752H, 30% | 20 | 218.7 | 1 | 18270.5 | 14.8 |
| **VH-024-001** | RG752H, 40% | 20 | 218.7 | 1 | 16406.1 | 13.3 |
| **Group 4** - **Salt** | Ca Pamoate | 5 | 19.6 | 8 | 3444.0 | 11.1 |
| **Group 5** - **Salt** | Na Oleate | 5 | 17.5 | 8 | 3331.9 | 10.8 |

### EXAMPLE 5

### In Vitro Cetrorelix Release Studies of Additional Microsphere Formulations

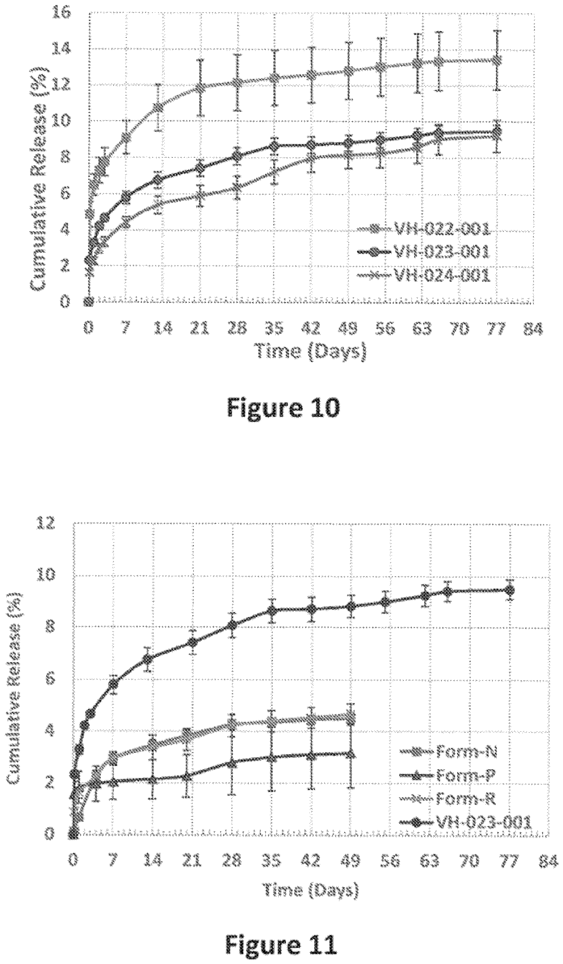

In order to supplement the *in vivo* pharmacokinetic data and further optimize the compositions of cetrorelix-loaded microspheres *in vitro* release studies were carried out wherein 45 mg of microspheres were incubated in 0.5 ml Tris Mannitol, pH 7.4 at 37°C. The results for *in vitro* release for the salt-free formulations used in pharmacokinetic studies are summarized in **Figure 10****.** In contrast to the *in vivo* data, the increase of PLGA content in the composition from 20% to 30% resulted in marked reduction of cetrorelix release rate and of cumulative release. Surprisingly, further increase of PLGA content from 30% to 40%, while resulting in further decrease in release rate, also yielded negligible change in cumulative release levels, especially in the long term. Moreover in all formulations the cumulative release appears to plateau after about 60 days suggesting that the maximum cumulative release has been achieved.

In order to further explore the effect of salt and polymer on cetrorelix release, several formulations of salt-containing 15% RG502H (50:50 lactide:glycolide) and 15% RG752H (75:25 lactide:glycolide) (30% total PLGA content) cetrorelix-loaded microspheres (Table 7) were tested and compared with salt-free formulations.

**Table 7 Salt-containing RG502H/RG752H (30% PLGA) cetrorelix-loaded microsphere formulations**

| **ID** | **Salt** | **Cetrorelix Salt (mg)** | **Cetrorelix Content (%,w/w)** | **PLGA* (mg)** | **NMP (mg)** | **Total (mg)** | **Cetrorelix Loading (%,w/w)** | **PLGA Solution (%,w/w)** | **Total Solid (%,w/w)** |
|---|---|---|---|---|---|---|---|---|---|
| **Form-N** | Ca Pamoate | 63.29 | 77% | 135.0 | 316.3 | 514. 6 | 9.5 | 29.9 | 38.5 |
| **Form-P** | Na Oleate | 58.80 | 83% | 134.7 | 317.4 | 510. 9 | 9.6 | 29.8 | 37.9 |
| **Form-R** | Ca Citrate | 62.20 | 78% | 134.9 | 316.5 | 513. 6 | 9.4 | 29.9 | 38.4 |

| | | | | | | | | | |
|---|---|---|---|---|---|---|---|---|---|
| ^{∗} PLGA = 50:50 RG752H:RG502H (w:w) | | | | | | | | | |

The results show that the presence of salt markedly decreases cumulative cetrorelix release levels. While Na Oleate was particularly effective **(****Figure 11****),** all salts resulted in lower cumulative release compared to corresponding salt free concentration. In addition, cetrorelix cumulative release appeared to reach plateau after 49 days. At the same time Na Oleate and Ca Citrate did not appear to have any effect on the initial release rate while it was slightly decreased in the presence of Ca Pamoate.

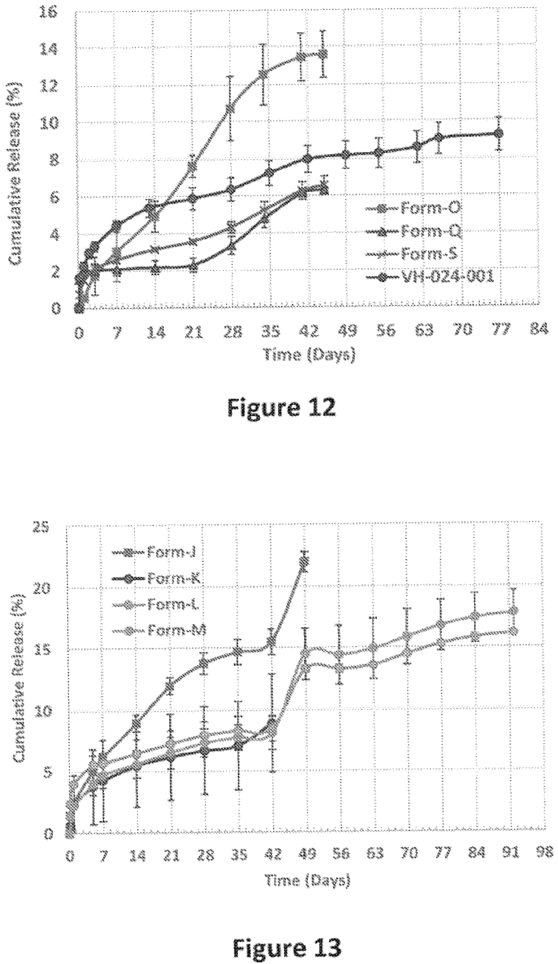

Surprisingly, when the polymer content was increased to 40% and replaced with RG752H (75:25 lactide:glycolide) **(Table 8)** the presence of Ca Pamoate slowed the initial release rate but dramatically (nearly 3-fold) increased cumulative cetrorelix release, as compared with salt free formulation. On the other hand, similarly to RG502H/RG752H data, Na Oleate and Ca Citrate did not appear to have any effect on the initial release rate although these formulations displayed a modest increase in cumulative release levels **(****Figure 12****).**

**Table 8 Salt-containing RG752H (40% PLGA) Cetrorelix-loaded microsphere formulations**

| **ID** | **Salt** | **Cetrorelix Salt (mg)** | **Cetrorelix Content (%,w/w)** | **RG752H (mg)** | **NMP (mg)** | **Total (mg)** | **Cetrorelix Loading (%,w/w)** | **PLGA Solution (%,w/w)** | **Total Solid (%,w/w)** |
|---|---|---|---|---|---|---|---|---|---|
| **Form-O** | Ca Pamoate | 62.95 | 77% | 180.5 | 268.4 | 511.8 | 9.5 | 40.2 | 47.6 |
| **Form-Q** | Na Oleate | 58.53 | 83% | 180.6 | 269.4 | 508.6 | 9.6 | 40.1 | 47.0 |
| **FormS** | Ca Citrate | 56.88 | 78% | 166.2 | 246.5 | 469.5 | 9.4 | 40.3 | 47.5 |

In an attempt to investigate the effect of polymer and N-methyl-2-pyrrolidone (NMP) on cetrorelix release rate and cumulative release, microsphere formulations using 20% RG502H and 20% RG752H, or 40% RG752H, with and without NMP **(Table 9)** were tested.

**Table 9 RG502H/RG752H (40% PLGA) Cetrorelix-loaded microsphere formulations with and without NMP.**

| **ID** | **Cetrorelix (mg)** | **RG752S (mg)** | **RG502H + RG752H (mg)*** | **NMP (mg)** | **DMSO (mg)** | **Total (mg)** | **Cetrorelix Loading (%,w/w)** | **PLGA Solution (%,w/w)**** | **Total Solid (%,w/w )** |
|---|---|---|---|---|---|---|---|---|---|
| Form-J | 45.3 | 167.43 | 0 | 257 | 0 | 470.1 | 9.6 | 39.4 | 45.3 |
| Form-K | 45.4 | 168.55 | 0 | 0 | 251 | 465.2 | 9.8 | 40.1 | 46.0 |
| Form-L | 45.6 | 0 | 166.98 | 252 | 0 | 464.2 | 9.8 | 39.9 | 45.8 |
| Form-M | 45.9 | 0 | 169.87 | 0 | 252 | 467.3 | 9.8 | 40.3 | 46.2 |

| | | | | | | | | | |
|---|---|---|---|---|---|---|---|---|---|
| ^{∗} RG502H:RG752H (w:w) = 50:50 ^{∗∗} PLGA (%,w/w) = PLGA wt ÷ (PLGA wt + Total Solvent wt) | | | | | | | | | |

The results are summarized in **Figure 13** and **Table 10.** Surprisingly, omitting NMP from 40%RG752H resulted in dramatic decrease in both cetrorelix release rate and total release. Interestingly, 20% RG502H / 20% RG752H microspheres displayed NMP-independent multiphasic cetrorelix release profile consisting of initial burst phase followed by a first plateau phase at about 8% cumulative release, achieved on day 42, followed by the second burst phase on days 42-49, and subsequently followed by a second plateau phase, at approximately 17% cumulative release, achieved on day 92.

**Table 10 In vitro Cetrorelix release by 40% RG752H - microsphere formulations with and without NMP**

| **ID** | **Total Release (%)** | **Residual Loading (%)** | **Total Recovery (%)** |
|---|---|---|---|
| **Form-J** | 22.0 (743.3 µg) | 89.0 | 111.0 |
| **Form-K** | 11.1 (362.0 µg) | 89.4 | 100.2 |

It will be appreciated by those skilled in the art that changes could be made to the embodiments described above without departing from the broad inventive concept thereof. It is understood, therefore, that this invention is not limited to the particular embodiments disclosed, but it is intended to cover modifications that are within the spirit and scope of the invention, as defined by the appended claims.

## Claims

1. A long-term drug release composition comprising: a therapeutically effective amount of a GnRH antagonist in combination with a polymer, wherein said composition is capable of releasing said GnRH antagonist for a long term.

2. The composition of claim 1, wherein said composition is capable of
- releasing said GnRH antagonist for more than 90 days; or
- achieving a therapeutic effect within 24 hrs and maintains therapeutic effect for at least 90 days.

3. The composition of claim 1, wherein said composition is in the form of a hydrogel, a flowable composition, a microsphere, or an implant.

4. The composition of claim 1, wherein said GnRH antagonist is cetrorelix, abarelix, degarelix, ganirelix, ozarelix, taverelix, antarelix, or iturelix.

5. The composition of claim 1, wherein said polymer is poly(glycolide) (PLG), poly(lactide) (PLA), or poly-lactic co-glycolic acid (PLGA).

6. The composition of claim 1, wherein said polymer is a non-PLGA polymer; preferably wherein said non-PLGA polymer is poly ethyleneglycol (PEG), PLG, PLA, polybutylene terephthalate (PBT), poly(epsilon-caprolactone) (PCL), dioxanone, butanediisocyanate, butanediol, or a combination thereof.

7. A flowable composition, the composition comprising: (a) a biodegradable thermoplastic polyester that is substantially insoluble in aqueous medium or body fluid; (b) a biocompatible polar aprotic solvent, wherein the biocompatible polar aprotic solvent is miscible to dispersible in aqueous medium or body fluid; and (c) a therapeutically effective amount of a GnRH antagonist.

8. The composition of claim 7, wherein said flowable composition is capable of forming an implant in situ, after its administration into a subject.

9. The composition of claim 7, wherein said flowable composition is
- an injectable composition; or
- injectable intramuscularly or subcutaneously.

10. The composition of claim 7, wherein said biodegradable thermoplastic polymer is substantially insoluble in aqueous medium or body fluid,

11. The composition of claim 10, wherein the thermoplastic polyester is a polylactide, a polyglycolide, a polycaprolactone, a copolymer thereof, a terpolymer thereof, or any combination thereof.

12. The composition of claim 7, wherein the solvent is capable of diffusing into body fluid so that the flowable composition coagulates or solidifies.

13. The composition of claim 12, wherein the solvent is N-methyl-2-pyrrolidone, 2-pyrrolidone, N,N-dimethylformamide, dimethyl sulfoxide, propylene carbonate, caprolactam, triacetin, or any combination thereof.

14. The composition of claim 7, wherein said flowable composition comprises an Atrigel^{®} delivery system, said system comprising a copolymer, a water soluble organic solvent, and said GnRH antagonist.

15. A method of preparing a flowable composition of claim 7, the method comprising: mixing a biodegradable thermoplastic polymer, a biocompatible solvent; and a Gonadotropin-releasing hormone (GnRH) antagonist.
